# EUROPÄISCHE PATENTSCHRIFT

(11) **EP 0 778 303 B1**
(45) Veröffentlichungstag und Bekanntmachung des Hinweises auf die Patenterteilung: **03.07.2002**
(21) Anmeldenummer: 96114215.5
(22) Anmeldetag: 05.09.1996
(51) Int. Cl.: C08G 18/78, C08G 18/80, C09D 175/04, C07C 273/18

(54) **Allophanatgruppen aufweisende Polyisocyanate auf Basis Diphenylmethandiisocyanat mit überwiegend oder vollständig blockierten Isocyanatgruppen**
Polyisocyanates containing allophanate groups based on diphenylmethanediisocyanate and having predominantly or completely blocked isocyanate groups
Polyisocyanates contenant des groupes allophanate à base de diphénylméthanediisocyanate à groupes isocyanate bloqués en majorité ou complètement

(30) Priorität: 18.09.1995 DE 19534624
(43) Veröffentlichungstag der Anmeldung: 11.06.1997
(73) Patentinhaber: BAYER AG, 51368 Leverkusen (DE)
(72) Erfinder: Schmalstieg, Lutz, Dr., 50676 Köln (DE); Pedain, Josef, Dr., 51061 Köln (DE); Engbert, Theodor, 50968 Köln (DE); Casselmann, Holger, Dr., 51469 Bergisch Gladbach (DE); Kobelka, Frank, 51381 Leverkusen (DE)

(56) Entgegenhaltungen:
- EP-A- 0 000 016
- EP-A- 0 053 766
- EP-A- 0 649 866

## Beschreibung

Die Erfindung betrifft neue Polyisocyanate auf der Basis von Diphenylmethandiisocyanat mit mindestens 95 % blockierten Isocyanatgruppen, ein Verfahren zu ihrer Herstellung und ihre Verwendung als Vernetzer in Polyurethan-Einbrennlacken.

Die Verwendung von blockierten Polyisocyanaten zur Herstellung von thermisch härtbaren Polyurethanbeschichtungen ist bekannt (z.B. Kunststoffhandbuch, Bd. VII, Polyurethane, Carl Hanser Verlag, München 1966, Seite 11-13 und 21ff). Es sind eine Reihe von Verbindungen beschrieben, die sich zur reversiblen Blockierung von Isocyanaten eignen. Unter diesen kommt der Klasse der C-Haciden Verbindungen (Beispiele: Malonsäure- und Acetessigsäureester) aufgrund chemischer und physiologischer Eigenschaften eine besondere Bedeutung zu (vgl. z.B. DE-A 2 342 603, 2 436 872, 2 550 156, 2 612 783, 2 612 784, 2 612 785). In der DE-A 2550156 und DE-A 2639491 werden Polyurethaneinbrennlacke auf Basis von mit Malonestern oder Acetessigestern blockierten aliphatischen Polyisocyanaten und organischen Polyhydroxylverbindungen beschrieben. Gemäß der Lehre der DE-A 3 046 409 sind die Polyurethaneinbrennlacke auf Basis aliphatischer Polyisocyanate aber nicht zur Herstellung von Beschichtungen mit hoher Schlag- und Stoßfestigkeit geeignet. Zur Herstellung derartiger Beschichtungen ist die Verwendung von Polyisocyanaten der Diphenylmethanreihe gemäß DE-A 3 046 409 vorteilhaft.

Auch die Beschichtungssysteme der letztgenannten Veröffentlichung sind mit Nachteilen behaftet: die dort beschriebenen blockierten Polyisocyanatvernetzer zeigen eine ungenügende Kristallisationsbeständigkeit, weswegen sie mit einer Polyolkomponente abgemischt werden müssen. Durch diese Maßnahme werden die Eigenschaften der Beschichtungen soweit vorbestimmt, daß der Verarbeiter nur noch wenig Möglichkeiten hat das Beschichtungssystem dem jeweiligen Anwendungszweck anzupassen.

Zum zweiten härten die Beschichtungssysteme der DE-A 3 046 409 nach eigenen Versuchen unter Ausbildung einer leichten Oberflächenstruktur aus, weswegen solche Beschichtungen nicht optimal hochglänzend überlackierbar sind.

Aufgabe der Erfindung ist, verbesserte Diethylmalonat-blockierte Polyisocyanatvernetzer auf Basis Diphenylmethandiisocyanat zur Verfügung zu stellen, die sich durch gute Kristallisationsbeständigkeit einerseits und excellenten Verlauf auf dem Substrat andererseits auszeichnen. Diese Aufgabe wird mit der Bereitstellung der erfindungsgemäß blockierten Polyisocyanate gelöst.

Gegenstand der Erfindung sind Polyisocyanate auf Basis Diphenylmethandiisocyanat, deren Isocyanatgruppen zu mindestens 95% mit Malonsäurediethylester blockiert sind, dadurch gekennzeichnet, daß sie in lösemittelfreier Form
A) einen Gehalt an unblockierten oder blockierten Isocyanatgruppen (berechnet als NCO) von insgesamt 9% bis 13%
   und
B) einen Gehalt an Allophanatgruppen (berechnet als C2HN2O3, Molekulargewicht 101) von 3% bis 9%
   und
C) eine berechnete mittlere Funktionalität von 2,0 bis 2,5 aufweisen.

Gegenstand der Erfindung ist auch ein Verfahren zur Herstellung von organischen Polyisocyanaten, deren Isocyanatgruppen zu mindestens 95% mit Diethylmalonat blockiert sind, dadurch gekennzeichnet, daß Diphenylmethandiisocyanat in Gegenwart eines Katalysators mit Monoalkoholen gegebenenfalls im Gemisch mit untergeordneten Mengen an Diolen und anschließende Blockierung mit Diethylmalonat umgesetzt wird.

Gegenstand der Erfindung ist auch die Verwendung der erfindungsgemäßen Polyisocyanate mit überwiegend oder ausschließlich blockierten Isocyanatgruppen als Vernetzer für organische Polyhydroxylverbindungen in Polyurethaneinbrennlacken.

Die erfindungsgemäßen blockierten Polyisocyanate sind Umsetzungsprodukte von überschüssigem Diphenylmethandiisocyanat mit Monoalkoholen, gegebenenfalls im Gemisch mit geringen Mengen an Diolen.

Als Ausgangsdiisocyanat ist 4,4'-Diphenylmethandiisocyanat geeignet, gegebenfalls im Gemisch mit bis zu 60 Gew.-% 2,4'-Diphenylmethandiisocyanat und weniger als 6 Gew.-% 2,2'-Diphenylmethandiisocyanat. Die Verwendung von technischem 4,4'-Diphenylmethan-diisocyanat, enthaltend weniger als 2 Gew.-% 2,4'-Diphenylmethandiisocyanat und weniger als 0,5 Gew.-% an 2,2'-Diphenylmethandiisocyanat ist bevorzugt.

Das eingesetzte Diphenylmethandiisocyanat wird vor der Blockierung teilweise zum Allophanat umgesetzt durch katalysierte Umsetzung mit Monoalkoholen, gegebenenfalls im Gemisch mit geringen Mengen an Diolen. Das NCO/OH Verhältnis beträgt 5:1 bis 12:1.

Als Alkoholkomponente werden Monoalkohole mit 2 bis 22, vorzugsweise 2 bis 10 Kohlenstoffatomen eingesetzt, die gegebenenfalls zusätzlich Ether- und/oder Estergruppen enthalten können. Ganz besonders bevorzugt werden bei Raumtemperatur flüssige, Ethergruppen-freie, einwertige Alkohole mit 4 bis 10 Kohlenstoffatomen eingesetzt.

Geeignete Monoalkohole sind beispielsweise Ethanol, n-Propanol, i-Propanol, n-Butanol, i-Butanol, n-Hexanol, 2-Ethylhexanol, n-Octanol, n-Decanol, n-Dodecanol, n-Hexadecanol, oder Milchsäureethylester.

Erfindungsgemäß einsetzbar sind auch Ethergruppen aufweisende einwertige Alkohole, wie sie durch Ethoxylierung und/oder Propoxylierung der obengenannten einwertigen Alkohole erhalten werden, wie zum Beispiel Diethylenglykolmonobutylether. Es können auch Mischungen der genannten Alkoholkomponenten eingesetzt werden.

Gegebenenfalls können bei der Herstellung der Allophanate auch untergeordnete Mengen an Diolen mitverwendet werden. Geeignete Diole sind beispielsweise 1,2-Ethandiol, 1,2-Propandiol, 1,4- bzw. 1,3-Butandiol, 1,6-Hexandiol, 2-Ethylhexandiol-1,3, 1,8-Octandiol oder auch Dimerfettalkohole. Ebenfalls einsetzbar sind Ethergruppen aufweisende Diole wie z.B. Diethylenglykol, Triethylenglykol, Tetraethylenglykol, Dipropylenglykol, Tripropylenglykol oder auch Gemische derartiger Alkohole.

Die Menge an Diol die erfindungsgemäß mitverwendet werden kann beträgt in der Regel weniger als 80 Mol% der Menge an Monoalkohol, bevorzugt weniger als 50 Mol% der Menge an Monoalkohol. Ganz besonders bevorzugt wird ohne Zusatz von Diolen gearbeitet, so daß das entstehende Allophanat-gruppen-enthaltende Polyisocyanatgemisch difunktionell ist.

Im Rahmen der vorliegenden Erfindung wurde die Beobachtung gemacht, daß difunktionelle Produkte einen hervorragenden Verlauf der resultierenden Lacke auf dem Substrat ermöglichen.

Die Umsetzung des Diphenylmethan-diisocyanats mit der Alkoholkomponente erfolgt zumindest im Anschluß an die intermediäre Urethanbildung in Gegenwart von die Allophanatbildung fördernden Katalysatoren. Geeignete Katalysatoren sind beispielsweise in dem Reaktionsgemisch lösliche Metallverbindungen der 3. und 4. Hauptgruppe sowie der 1. 2. 6. und 8. Nebengruppe des Periodensystems der Elemente nach Mendeleijew (z.B. US-A 3 769 318). Besonders bevorzugt werden Zinn(II)octoat, Zinkstearat oder Zinkacetylacetonat in Mengen von 20 bis 2000 ppm (Gewicht), vorzugsweise 20 bis 200 ppm (Gewicht), bezogen auf das Reaktionsgemisch eingesetzt. Die Anwesenheit von alkylierend wirkenden Verbindungen während der Reaktion wie in der US-A 3 769 318 beschrieben ist weniger bevorzugt.

Die zur Herstellung der erfindungsgemäßen blockierten Polyisocyanatmischungen einzusetzenden Polyisocyanate enthalten ausschließlich Allophanatstrukturen und sind im wesentlichen frei von Urethan-, Isocyanurat-, und/oder Carbodiimidstrukturen. Dies wird durch ¹³C-NMR-spektroskopische Untersuchungen zweifelsfrei belegt.

Die Allophanatmodifizierung wird bei Temperaturen von bis zu 160°C durchgeführt. Die bevorzugte Temperatur bezüglich der Allophanatisierungsreaktion liegt bei 80 bis 120°C. Nach Erreichen des für die Allophanatbildung berechneten NCO-Gehaltes wird die Reaktion vorzugsweise durch Zugabe eines Katalysatorengiftes abgebrochen. Geeignete Katalysatoren-gifte sind beispielsweise alkylierend oder acylierend wirkende Verbindungen, wie p-Toluol-sulfonsäuremethylester, Dimethylsulfat, Benzoylchlorid oder Isophthalsäuredichlorid, die vorzugsweise in mindestens äquimolaren Mengen, bezogen auf die Menge des Katalysators, eingesetzt werden.

Die so erhaltenen Allophanatgruppen aufweisenden Polyisocyanatmischungen fallen als gelblich gefärbte niedrigviskose Flüssigkeiten mit einem NCO-Gehalt von 14,5% bis 28,5% an und werden in der zweiten Stufe des erfindungsgemäßen Verfahrens direkt in einer Blockierungsreaktion mit Malonsäurediethylester umgesetzt. Diese Blockierungsreaktion geschieht in an sich bekannter Weise (z.B. DE-A 2 342 603, DE-A 2 550 156) mit Hilfe basischer Katalysatoren, wie zum Beispiel Natriumphenolat, Natriummethylat oder anderen Alkalialkoholaten. Auch andere Alkaliverbindungen als Katalysator kommmen in Betracht wie Natriummalonat. Die Katalysatoren werden in einer Menge von 0,1% bis 2%, bezogen auf Gesamtgewicht der Reaktionskomponente, zugesetzt. Die Einsatzmenge an Diethylmalonat sollte mindestens 1 Mol pro Isocyanatäquivalent betragen; es ist jedoch zweckmäßig, einen 5 bis 20%igen Überschuß an Blockierungsmittel zu verwenden.

Die Blockierungsreaktion kann lösungsmittelfrei oder in Anwesenheit von gegenüber Isocyanaten inerten Lösungsmitteln erfolgen. Als solche kommen beispielsweise in Betracht: Ethylacetat, Methylglykolacetat, Ethylglykolacetat, Butylacetat, Methoxypropylacetat, Methylethylketon, Toluol, Xylol sowie die in der Lackchemie üblichen höhersiedenden Kohlenwasserstoffgemische.

Die vorgenannten Lösungsmittel können einzeln oder als Gemisch zum Einsatz kommen. Anstelle von Lösungsmitteln ist es auch möglich, Weichmacher zu verwenden, wie z.B. handelsübliche Phosphorsäureester, Phthalsäureester oder Sulfonsäureester.

Es ist bevorzugt, die Menge an Lösungsmitteln oder Weichmachern so zu bemessen, daß in den erfindungsgemäßen überwiegend oder vollständig blockierten Polyisocyanaten der Festkörpergehalt von 70 bis 90 Gew.-% beträgt.

Bei den erfindungsgemäßen überwiegend oder vollständig blockierten Polyisocyanaten handelt es sich um hochviskose oder harzartige Produkte, die sich durch hervorragende Kristallisationsbeständigkeit auszeichnen, insbesondere Produkte auf Basis von 4,4'-Diphenyl-methandiisocyanat.

Die erfindungsgemäßen überwiegend oder vollständig blockierten Polyisocyanate stellen wertvolle Vernetzerharze für Polyhydroxylverbindungen bei der Herstellung von Einbrennlacken dar. Geeignete Polyhydroxylverbindungen für diesen Einsatzzweck sowie weitere Details bezüglich der Herstellung und Anwendung derartiger Einbrennlacke können der einschlägigen Literatur, beispielsweise Z.W.Wicks, Progr. Org. Coat. 9, Seite 20 (Applications) 1981, entnommen werden.

Beschichtungen auf Basis der erfindungsgemäßen überwiegend oder vollständig blockierten Polyisocyanatvernetzer zeichnen sich durch exzellente Haftung, hohe Elastizität und gute Oberflächenhärte aus. Besonders hervorzuheben ist der im Vergleich zu den Systemen der DE-A 3 046 409 nochmals verbesserte Verlauf. Einbrennlacke auf Basis der erfindungsgemäßen überwiegend oder vollständig blockierten Polyisocyanate zeigen keinerlei Oberflächenstruktur. Aufgrund der genannten Eigenschaften sind die erfindungsgemäßen blockierten Polyisocyanate besonders geeignet zur Herstellung von Automobileinbrennfüllern.

Alle Angaben in Prozent oder Teilen beziehen sich auf das Gewicht.

### Beispiel 1

### Herstellung eines erfindungsgemäßen blockierten Polyisocyanats

937,5g technisches 4,4'-Diphenylmethandiisocyanat werden aufgeschmolzen. Zu der klaren Schmelze werden bei 80°C 74g n-Butanol zugetropft. Nach Abklingen der exothermen Reaktion setzt man 0,05g Zinkacetylacetonat zu und rührt bei 80°C ca. 5 Stunden. Danach ist die Allophanatmodifizierung abgeschlossen, das Reaktionsgemisch weist einen NCO-Gehalt von 22,5% auf. Nach Zusatz von 0,05g Isophthalsäuredichlorid gibt man 885g Malonsäurediethylester zu, wodurch sich das Reaktionsgemisch auf 50°C abkühlt. Bei dieser Temperatur tropft man eine Lösung von 3g einer 30%igen Natriummethylatlösung in Methanol in 87,5g Malonsäurediethylester langsam zu. Nach Abklingen der exothermen Reaktion rührt man 7 Stunden bei 50°C nach und verdünnt dann mit einer Mischung aus gleichen Teilen Butylacetat und ^{R}Solvesso 100 (aromatisches Kohlenwasserstoffgemisch der Firma Exxon Corp.). Der so erhaltene blockierte Polyisocyanatvernetzer ist bei 0°C kristallisationsstabil und weist folgende Kenndaten auf:

| | |
|---|---|
| Festkörpergehalt | 80% |
| Viskosität | 750 mPas (23°C) |
| freier NCO-Gehalt | 0,1% (bez. auf Lösung) |
| blockierter NCO-Gehalt | 9,2% (berechnet, bez. auf Lösung) |
| Funktionalität | 2 (berechnet) |
| Allophanatgruppengehalt | 5,1% (berechnet, bez. auf Festharz) |

### Beispiel 2

### Herstellung eines erfindungsgemäßen blockierten Polyisocyanates

Man verfährt wie in Beispiel 1, jedoch werden anstelle von n-Butanol 118g Milchsäureethylester eingesetzt.

Der erhaltene Polyisocyanatvernetzer ist bei 0°C kristallisationsstabil und weist nach entsprechender Verdünnung folgende Kenndaten auf:

| | |
|---|---|
| Festkörpergehalt | 80% |
| Viskosität | 1600 mPas (23°C) |
| freier NCO-Gehalt | ca. 0,1% (bez. auf Lösung) |
| blockierter NCO-Gehalt | 9,1% (berechnet, bez. auf Lösung) |
| Funktionalität | 2 (berechnet) |
| Allophanatgruppengehalt | 5,0% (berechnet, bez. auf Festharz) |

### Beispiel 3

### Herstellung eines erfindungsgemäßen blockierten Polyisocyanates

Man verfährt wie in Beispiel 1.
Eingesetzt werden:

| | |
|---|---|
| 1250g eines Gemisches aus | 82 % 4,4'-Diphenylmethandiisocyanat |
| | 17,5 % 2,4'-Diphenylmethandiisocyanat |
| | 0,5 % 2,2'-Diphenylmethandiisocyanat |
| 130 g 2-Ethylhexanol | |
| 1440g Diethylmalonat (Gesamtmenge) | |

Der erhaltene Polyisocyanatvernetzer ist bei einer Temperatur von 10°C kristallisationsstabil und weist nach entsprechender Verdünnung folgende Kenndaten auf:

| | |
|---|---|
| Festkörpergehalt | 80% |
| Viskosität | 2500 mPas (23°C) |
| freier NCO-Gehalt | nicht nachweisbar |
| blockierter NCO-Gehalt | 9,5 % (berechnet, bez. auf Lösung) |
| Funktionalität | 2 (berechnet) |
| Allophanatgruppengehalt | 3,6% (berechnet, bez. auf Festkörper) |

### Beispiel 4

### Herstellung eines Einbrennlackes auf Basis eines erfindungsgemäßen Polyisocyanatvernetzers wie er bei der Automobilerstlackierung im Füllerbereich eingesetzt werden kann

135,3 Teile einer Polyester-Lösung, 65%ig in einem Lösungsmittelgemisch aus ^{R}Solvessol/100 i-Butanol im Gewichtsverhältnis 31,5:3,5 mit einem Hydroxylgehalt von 1,7% (berechnet als OH), der Säurezahl von 5 und einer Viskosität von 2600 mPas (23°C) (^{R}Alkynol 1665, Handelsprodukt der Bayer AG) werden mit 180 Teilen Bariumsulfat (Blanc fixe micro der Sachtleben-Chemie, Duisburg), 60 Teilen der Farbpigmentmischung RAL 7000 bestehend aus 54,78 Teilen Titandioxid (R-KB-4 der Bayer AG), 3,84 Teilen Eisenoxidpigment (Bayferrox 130 F der Bayer AG), 0,9 Teilen eines Farbpigmentes (Heliogenblau L7101 F der BASF AG, Ludwigshafen), und 0,48 Teilen eines Eisenoxidpigmentes (Bayferrox 3910 F der Bayer AG), 1,5 Teilen eines Dispergierhilfsmittels (Typ: Antiterra U, 50%ige Lösung, der Byk-Chemie,Wesel), 0,3 Teilen Kieselgel (Aerosil R 972 der Firma Degussa, Frankfurt/Main), 40,3 Teilen 1-Methoxypropylacetat-2, 40,3 Teilen Butylacetat und 40,3 Teilen Xylol versetzt und 45 Minuten in einer Perlmühle unter Kühlung dispergiert, wobei die Temperatur des Mahlansatzes 50°C nicht überschreiten sollte.

Die erhaltene Paste wird mit weiteren 108,3 Teilen einer Polyesterlösung, 80%ig in Butylacetat, mit einem Hydroxylgehalt von 3,4% (berechnet als OH), der Säurezahl 1 und einer Viskosität von 3000 mPas (23°C) (^{R}Desmophen 670, Handelsprodukt der Bayer AG), 3 Teilen eines Verlaufsmittels (Byk 358, 50%ige Lösung, der Byk-Chemie, Wesel), 160,5 Teilen des Polyisocyanatvernetzers aus Beispiel 1 versetzt und unter Rühren mit 14 Teilen 1-Methoxypropylacetat-2, 14 Teilen Butylacetat und 14 Teilen Xylol verdünnt.

Der so erhaltene Spritzlack läßt sich gut applizieren und zeigt einen hervorragenden Verlauf.

Der auf mit Elektrotauchlack beschichteten Blechen ausgehärtete Film zeigt keinerlei Oberflächenstruktur. Es lassen sich blasenfreie, oberflächenstörungsfreie Trockenfilmdicken >45 µm in einem Arbeitsgang herstellen. Der Lack härtet bereits bei einer Einbrenntemperatur von 130°C aus. Die optimale Einbrenntemperatur für diesen Lack beträgt 140°C.

Physikalischen Eigenschaften des Lackfilms nach 30 minütigem Einbrennen bei 130°C:

| | |
|---|---|
| Pendelhärte nach König (DIN 53117) | 152 sec |
| Erichsen Tiefung (DIN 53156) | 10,0 mm |
| Impact-Test (ASTM D 2794). | >80 i/p |

Im Lackaufbau zeigt der Füller gute Haftung zum Untergrund (Elektrotauchlackierung) und gute Zwischenhaftung zu Basis- und Decklackierungen.

Im Multischlagtest nach VDA (Verband der Automobilindustrie, Modell 508, 2 mal 500g Stahlschrot, 1,5 bar) gemäß festgelegtem Auswerteschema findet man eine hohe Steinschlagfestigkeit.

### Beispiel 5

### Nicht erfindungsgemäßes Vergleichsbeispiel im Hinblick auf DOS 3046409

Man verfährt wie in Beispiel 1, verzichtet jedoch auf eine Allophanatmodifizierung.

Eingesetzt werden:
1250g 4,4'-Diphenylmethandiisocyanat
1760g Diethylmalonat (Gesamtmenge)

Der erhaltene blockierte Polyisocyanatvernetzer weist nach entsprechender Verdünnung folgende Kenndaten auf:

| | |
|---|---|
| Viskosität | 1400 mPas (23°C) |
| freier NCO-Gehalt | 0,15% (bez. auf Lösung) |
| blockierter NCO-Gehalt | 11,1% (berechnet, bez. auf Lösung) |

Das Produkt kristallisiert nach einer Lagerdauer von 7 Tagen bei Raumtemperatur aus.

## Patentansprüche

1. Polyisocyanate auf Basis von Diphenylmethandiisocyanat, deren Isocyanatgruppen zu mindestens 95% mit Malonsäurediethylester blockiert sind, **dadurch gekennzeichnet, daß** sie in lösemittelfreier Form
A) einen Gehalt an unblockierten oder blockierten Isocyanatgruppen (berechnet als NCO) von insgesamt 9% bis 13%
und
B) einen Gehalt an Allophanatgruppen (berechnet als C2HN2O3, Molekulargewicht 101) von 3% bis 9%
und
C) eine berechnete mittlere Funktionalität von 2,0 bis 2,5 aufweisen.

2. Polyisocyanate gemäß Anspruch 1, **dadurch gekennzeichnet, daß** sie auf Basis von 4,4'-Diphenylmethandiisocyanat hergestellt werden.

3. Polyisocyanate gemäß Anspruch 1, **dadurch gekennzeichnet, daß** sie eine mittlere berechnete Funktionalität von 2 aufweisen.

4. Verfahren zur Herstellung von Polyisocyanaten mit überwiegend oder ausschließlich blockierten Isocyanatgruppen, gegebenenfalls in in Lacklösungsmitteln gelöster Form gemäß Anspruch 1, **dadurch gekennzeichnet, daß** man Diphenylmethandiisocyanat unter intermediärer Urethanbildung mit einwertigen Alkoholen mit 2 bis 22 Kohlenstoffatomen gegebenenfalls unter Zusatz von untergeordneten Mengen an zweiwertigen Alkoholen unter Einhaltung eines NCO/OH-Verhältnisses von 5:1 bis 12:1 bei bis zu 160°C liegenden Temperaturen zur Reaktion bringt, wobei man die Umsetzung spätestens nach erfolgter Urethanbildung in Gegenwart eines die Allophanatbildung fördernden Katalysators durchführt und anschließend die freien Isocyanatgruppen durch Umsetzung mit Malonsäurediethylester blockiert.

5. Verwendung der organischen Polyisocyanate mit überwiegend oder ausschließlich blockierten Isocyanatgruppen gemäß Anspruch 1 als Vernetzer für organische Polyhydroxylverbindungen in Polyurethaneinbrennlacken.

6. Verwendung der organischen Polyisocyanate mit überwiegend oder ausschließlich blockierten Isocyanatgruppen gemäß Anspruch 1 als Vernetzer für Automobilfüller.

## Claims

1. Polyisocyanates based on diphenylmethane diisocyanate, the isocyanate groups of which are blocked to the extent of at least 95% by diethyl malonate, **characterised in that**, in solvent-free form, they have
A) a content of unblocked or blocked isocyanate groups (calculated as NCO) of 9% to 13% in all
and
B) a content of allophanate groups (calculated as C₂HN₂O₃, molecular weight 101) of 3% to 9%
and
C) a calculated average functionality of 2.0 to 2.5.

2. Polyisocyanates according to claim 1, **characterised in that** they are produced from 4,4'-diphenylmethane diisocyanate.

3. Polyisocyanates according to claim 1, **characterised in that** they have an average calculated functionality of 2.

4. Process for producing polyisocyanates containing predominantly or exclusively blocked isocyanate groups, optionally in a form dissolved in coating solvents, according to claim 1, **characterised in that** diphenylmethane diisocyanate is reacted at temperatures of up to 160°C with monohydric alcohols having 2 to 22 carbon atoms, optionally with addition of minor quantities of dihydric alcohols while maintaining an NCO/OH ratio of 5:1 to 12:1, with intermediate formation of urethane, and the conversion is carried out in the presence of a catalyst which promotes the formation of allophanate, at the latest after urethane formation is complete, and the free isocyanate groups are subsequently blocked by reaction with diethyl malonate.

5. Use of the organic polyisocyanates containing predominantly or exclusively blocked isocyanate groups according to claim 1 as cross-linking agents for organic polyhydroxy compounds in polyurethane stoving lacquers.

6. Use of the organic polyisocyanates containing predominantly or exclusively blocked isocyanate groups according to claim 1 as cross-linking agents for automobile fillers.

## Revendications

1. Polyisocyanates à base de diphénylméthanediisocyanate et dont les groupes isocyanate sont bloqués à au moins 95 % par le malonate de diéthyle, **caractérisés en ce que**, en l'absence de solvant, ils ont
A) une teneur en groupes isocyanate bloqués ou non (exprimé en NCO) de 9 à 13 % au total
et
B) ils ont une teneur en groupes allophanate (exprimé en C₂HN₂O₃, poids moléculaire 101) de 3 % à 9 %.
et
C) ils ont une fonctionnalité moyenne calculée de 2,0 à 2,5.

2. Polyisocyanates selon revendication 1, **caractérisés en ce qu'**ils sont préparés à base de 4,4'-diphénylméthanediisocyanate.

3. Polyisocyanates selon revendication 1, **caractérisés en ce qu'**ils ont une fonctionnalité moyenne calculée de 2.

4. Procédé pour la préparation de polyisocyanates à groupes isocyanate bloqués en majorité ou en totalité, le cas échéant à l'état dissous dans des solvants, selon revendication 1, **caractérisé en ce que** l'on fait réagir le diphénylméthanediisocyanate, avec formation intermédiaire d'huréthane, avec des alcools monovalents en C₂-C₂₂ éventuellement accompagnés de proportions mineures d'alcools divalents, en maintenant un rapport NCO/OH de 5:1 à 12:1, à des températures allant jusqu'à 160°C, en réalisant la réaction, au plus tard après formation d'uréthane, en présence d'un catalyseur favorisant la formation des allophanates, après quoi on bloque les groupes isocyanates libres par réaction avec le malonate de diéthyle.

5. Utilisation des polyisocyanates organiques à groupes isocyanate bloqués en majorité ou en totalité selon revendication 1, en tant qu'agents réticulants pour des composés organiques polyhydroxylés dans des vernis de polyuréthanne à cuire au four.

6. Utilisation des polyisocyanates organiques à groupes isocyanate bloqués en majorité ou en totalité selon revendication 1 en tant qu'agents réticulants pour des mastics automobiles. Le A 31 348 (96 114 215.5)
